# NEUE EUROPÄISCHE PATENTSCHRIFT

(11) **EP 0 316 518 B2**
(45) Veröffentlichungstag und Bekanntmachung des Hinweises auf die Entscheidung über den Einspruch: **29.10.1997**
(45) Hinweis auf die Patenterteilung: 08.03.1995
(21) Anmeldenummer: 88113096.7
(22) Anmeldetag: 12.08.1988
(51) Int. Cl.: A61F 13/15, A61L 15/46, A61L 15/60

(54) **Hygienischer Zellstoffartikel**
Sanitary cellulose article
Article hygiénique en cellulose

(30) Priorität: 13.11.1987 DE 3738601
(43) Veröffentlichungstag der Anmeldung: 24.05.1989
(73) Patentinhaber: VP - SCHICKEDANZ AG, D-90022 Nürnberg (DE)
(72) Erfinder: Hess, Jürgen, Dr., D-8500 Nürnberg 20 (DE); Petranyi, Pal, Dr., D-8500 Nürnberg 90 (DE)
(74) Vertreter: Hübner, Gerd, Dipl.-Phys.

(56) Entgegenhaltungen:
- EP-A- 0 050 375
- EP-A- 0 071 063
- EP-A- 0 138 179
- EP-A- 0 202 127
- EP-A- 0 205 674
- DE-A- 2 612 846
- FR-A- 2 513 875
- US-A- 3 669 103
- US-A- 3 794 034

## Beschreibung

Die Erfindung betrifft einen hygienischen Zellstoffartikel wie beispielsweise eine Windel, eine Krankenunterlage, eine Damenbinde, eine Slipeinlage oder dergl.. Derartige Zellstoffartikel sind bekannt; sie weisen ein oder mehrere Saugkissen auf, welche aus Zellstoff bestehen und in aller Regel Zusätze an Quellstoffen und gegebenenfalls Hilfsmaterialien haben. Die Artikel sind meist mit einer oberen flüssigkeitsdurchlässigen Abdeckung ausgerüstet und sie haben eine untere flüssigkeitsdichte Schicht oder Einlage. Die obere flüssigkeitsdurchlässige Abdeckung kann auch entfallen, wenn zumindest die obere Schicht des Saugkörpers derart verfestigt ist, daß der Saugkörper nicht mehr flusen kann. Eine solche Verfestigung kann durch Zusatz von thermoplastischen Fasern und anschließender zumindest oberflächlicher Wärmebehandlung herbeigeführt werden.

Auch die untere flüssigkeitsdichte Schicht oder Einlage kann notfalls entfallen; sie wird aber in den meisten Fällen vorhanden sein.

Der Zusatz der erwähnten Quellstoffe hat den Zweck, das Flüssigkeitsaufnahmevermögen des Artikels zu vergrößern. Als Quellstoffe werden heute meist synthetisch hergestellte Polymere verwendet, welche in der Lage sind, unter Vergrößerung ihres Volumens, erhebliche Mengen Flüssigkeit aufzunehmen. Es kommen hierzu Stoffe wie Polyacrylate, Pfropfpolymerisate aus Stärke und Acrylsäure oder ähnliche Verbindungen in Betracht, welche in der Literatur eingehend beschrieben sind. Als Fundstellen werden beispielsweise die amerikanischen Patentschriften US-A-4 382 919, USA 3 964 486 sowie US-A-3 669 103 genannt. Eine weitere Fundstelle ist die europäische Patentanmeldung EP-A-202 167.

Hygienische Zellstoffartikel der hier genannten Art weisen gegebenenfalls auch noch Hilfsmaterialien auf; hierunter sind solche Stoffe zu verstehen, die entweder den als Saugstoff wirkenden Zellstoff ganz oder teilweise ersetzen sollen, wie beispielsweise bestimmte Schaumstoffe, insbesondere hydrophile Polyurethanschäume oder die den Zweck haben, den pH-Wert des Saugkissens, der sich bei Benetzung mit Wasser oder physiologischen Flüssigkeiten einstellt, in den leicht sauren Bereich zu verschieben. Es hat sich dies als vorteilhaft erwiesen, da im sauren Bereich das Wachstum der meisten Mikroorganismen gehemmt oder unterbunden ist, so daß die Windel durch diese Zusätze zumindest für eine begrenzte Zeit gegen den Abbau der in ihnen gespeicherten Körperflüssigkeiten stabilisiert ist. Der Zusatz derartiger saurer Hilfsstoffe ist in der bereits genannten europäischen Patentanmeldung EP-A-202 127 beschrieben, aber auch in der deutschen Offenlegungsschrift DE-A-20 27 809.

Es sind bereits zahlreiche Versuche unternommen worden, hygienische Zellstoffartikel sowohl mit pH-Wert-regulierenden Stoffen, beispielsweise Zitronensäure als auch mit Quellstoffen auszurüsten. Dabei hat sich jedoch gezeigt, daß das Flüssigkeitsaufnahmevermögen der bekannten Quellstoffe stark erniedrigt wird, wenn der pH-Wert der Saugkörper in denjenigen Bereich gerückt wird, der aus physiologischen Gründen erforderlich ist, nämlich in den pH-Bereich unter 6,0, vorzugsweise in den Bereich von 5,0 - 6,0. Um den dadurch resultierenden Schwierigkeiten auszuweichen, wird in der europäischen Patentanmeldung EP-A-202 127 vorgeschlagen, den Saugkörperbereich eines derartigen Artikels in zwei Bereiche zu unterteilen, von denen einer die erwünschten Quellstoffe enthält, dafür aber frei von pH-Wert-regulierenden Zusätzen ist und von denen die übrigen Bereiche quellstofffrei sind, statt dessen aber pH-Wert-regulierende Stoffe aufweisen.

Es liegt auf der Hand, daß ein derartiger Aufbau eines hygienischen Zellstoffartikels, beispielsweise einer Windel, technisch aufwendig ist und das Produkt verteuert. Als weiterer Nachteil ist zu nennen, daß das Produkt an denjenigen Stellen an denen sich die Bereiche überlappen auch dicker wird, was zu einer Minderung des Tragekomforts führt. Am wichtigsten ist indes der Umstand, daß der pH-gepufferte Bereich nur auf den quellstofffreien Bereich, in dem sich die Puffer-Substanz befindet, beschränkt ist.

In der FR-A-2,513,875 werden Puffersubstanzen eingesetzt, die den pH-Wert auf ≦ 4,5 einstellen. Diese Puffersubstanzen wirken aber nicht als Quellstoffe.

Der Erfindung liegt die Aufgabe zugrunde, die bekannten hygienischen Zellstoffartikel, wie Windeln, Krankenunterlagen, Damenbinden, Slipeinlagen oder dergl. dahingehend weiter zu verbessern, daß sie sowohl die erwünschten antimikrobiellen Eigenschaften durch Stabilisierung im leicht sauren pH-Wert-Bereich aufweisen als auch über gute Saug- und Flüssigkeitsspeicher-Eigenschaften durch Zusatz von Quellstoffen verfügen. Die Lösung dieser Aufgabe ist durch die Beobachtung möglich geworden, daß es gelingen kann, die Quellstoffe selbst, welche chemisch betrachtet polymere Säuren sind, als Puffersubstanzen einzusetzen. Sie müssen hierzu mit Alkalimetallen und/oder Ammoniak derart teilneutralisiert werden, daß sich im Zellstoff-Saugkörper bei Benetzung mit Wasser oder einer physiologischen Flüssigkeit der erforderliche pH-Wert zwischen 5,0 und 6,0, vorzugsweise zwischen 5,2 und 5,5 einstellt. Überraschenderweise hat sich dabei ergeben, daß an dem bestehenden Vorurteil in zahlreichen Fällen ganz zu Unrecht festgehalten wird und daß das Flüssigkeitsspeichervermögen, aber auch das Flüssigkeitsansaugvermögen gewisser synthetischer Quellstoffe unter diesen Bedingungen praktisch durchaus hinreichende Werte aufweist.

Als besonders geeignet für diesen Zweck haben sich Quellstoffe erwiesen, welche Propfpolymere sind mit
0,5 - 20 Gewichtsprozent chemisch gebundenen Resten der allgemeinen Formel I,
79 - 99 Gewichtsprozent chemisch gebundenen Resten der allgemeinen Formel II, und
0,1 - 2 Gewichtsprozent chemisch gebundenen Resten eines Vernetzers, der aus Monomeren mit wenigstens 2 olefinisch ungesättigten Doppelbindungen hervorgegangen ist;
wobei bedeuten: 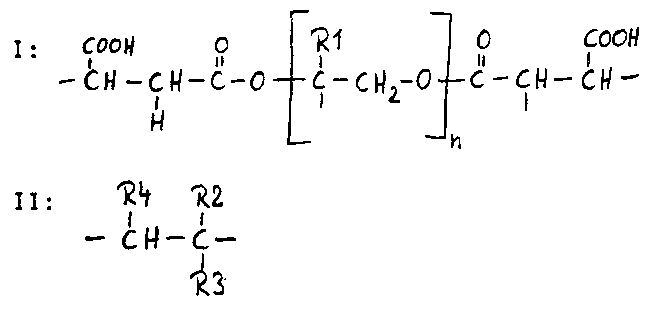
- n: = 2 bis 300
- R1: = Wasserstoff oder Methyl
- R2: = Wasserstoff, Methyl oder Ethyl
- R3: = Carboxyl, Sulfonyl, Phosphonyl, gegebenenfalls verestert mit Alkanol mit 1 - 4 C-Atomen oder eine Gruppe der Formel 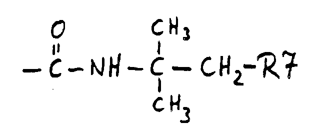 worin R7 eine Sulfonyl- oder Phosphonyl-Gruppe bedeuten,
- R4: = Wasserstoff, Methyl, Ethyl oder Carboxyl.

Pfropfpolymere dieser Art lassen sich unter Verwendung von Alkalimetallen oder Ammonium teilneutralisieren und stellen dann Puffersysteme dar, welche die Saugkörper von hygienischen Zellstoffartikeln auf beliebige Werte zwischen 5,0 und 6,0 einzustellen vermögen. Es ist leicht möglich, mit diesen Stoffen pH-Werte von 5,2 oder 5,5 einzustellen, derart, daß diese Werte auch über längere Zeit beim Gebrauch der Zellstoffartikel beibehalten werden. Die Quellstoffe werden, wie andere Quellstoffe auch, in Mengen von beispielsweise 10 - 50 % dem Zellstoff der hygienischen Zellstoffartikel beigemischt. Selbstverständlich ist es auch möglich, die Saugkörper der Zellstoffartikel nur teilweise, beispielsweise nur zonenweise mit diesen Quellstoffen zu versetzen; in jedem Fall tritt die erwähnte Wirkung ein, in denjenigen Bereichen, in denen sich die Quellstoffe befinden.

Die erwähnten Pfropfpolymerisate können beispielsweise durch Polymerisation in wässriger Lösung nach dem Verfahren der Gel-Polymerisation hergestellt werden. Dabei werden 15 - 50 %ige wässrige Lösungen der Comonomeren mit bekannten geeigneten Katalysator-Systemen ohne mechanische Durchmischung polymerisiert. Als Beispiel für die Herstellung eines solchen Pfropfpolymerisates wird die folgende Arbeitsanleitung gegeben:
In einem isolierten Polyethyleneimer von 10 Liter Inhalt werden 5 169 g E-Wasser vorgelegt, 1 000 g Natriumbicarbonat darin dispergiert und langsam 1 888 g Acrylsäure so zudosiert, daß die Reaktionslösung nicht überschäumt. Die Temperatur der Lösung kühlt sich dabei auf 12 - 10 °C ab. Es werden anschließend 100 g des unten angegebenen Umsetzungsproduktes, welches als Pfropfgrundlage dient, zugesetzt. Des weiteren werden 12 g Trimethylolpropantriacrylat und 10 g eines Natriumdiisooctylsulfosuccinates (Revopol V 21 33 der Firma Revo, Steinau) zugegeben. Bei einer Temperatur von 10-12 °C wird als Initiatoren ein Redox-System, bestehend aus 2,2 g 2,2'-Azobisamidinopropan-Dihydrochlorid, gelöst in 20 g Wasser, 4,4 g Kaliumperoxodisulfat, gelöst in 170 g Wasser und 6 g Natriumpyrosulfit, gelöst in 120 g Wasser, nacheinander zugegeben und verrührt. Die Reaktionslösung wird daraufhin ohne Rühren stehengelassen, wobei durch Einsetzen der Polymerisation die Temperatur bis auf 85 °C ansteigt. Es entsteht als Reaktionsprodukt ein festes Gel. Dieses wird anschließend mechanisch zerkleinert und bei Temperaturen über 80 °C getrocknet und gemahlen.

Als Pfropfgrundlage dient ein Produkt, welches wie folgt hergestellt wird:
In 345 g eines Blockcopolymeren aus 1,6 mol Propylenoxyd und 0,2 mol Ethylenoxyd mit einer OH-Zahl von 65 werden unter Rühren bei Raumtemperatur 39,2 g Maleinsäureanhydrid eingetragen und diese Mischung unter Rühren auf 80 °C erhitzt. Das Maleinsäureanhydrid löst sich dabei unter schwach exothermer Reaktion auf, es entsteht eine klare, schwach gelblich gefärbte Lösung.

Pfropfpolymerisate dieser Art haben den zusätzlichen Vorteil, daß sie unter den genannten Bedingungen Gele mit erhöhter Gelfestigkeit bilden, was auf den höheren Vernetzungsgrad dieser Stoffe gegenüber den meisten vorbekannten Quellstoffen zurückzuführen ist. Speziell auch diese höhere Gelfestigkeit ist in hygienischen Zellstoffartikeln vorteilhaft, da die Quellstoffe bei Wasseraufnahme festere Gallerten bilden und weitaus weniger zur Klebrigkeit neigen als vorbekannte Quellstoffe.

Wie gesagt, ist es auf die geschilderte Weise möglich, die gewünschten pH-Werte im Saugstoff von hygienischen Zellstoffartikeln leicht und sicher einzustellen und auch über die Gebrauchszeit der Zellstoffartikel zu halten. Dennoch kann es zur Erhöhung der Sicherheit erwünscht sein, den Zellstoffartikeln noch weitere Hilfsstoffe zuzufügen, welche den eingestellten und erwünschten pH-Wert weiter stabilisieren. Als Hilfsstoffe dieser Art können organische oder anorganische Monomere mit wenigstens 2 Säuregruppen eingesetzt werden, die mit Alkalimetallen oder Ammoniak derart teilneutralisiert sind, daß sich bei Benetzung des Saugkörpers mit Wasser oder physiologischer Flüssigkeit ein pH-Wert zu je 5,0 und 6,0, vorzugsweise von 5,2 - 5,5, einstellt. Als anorganisches Monomer kommt insbesondere Phosphorsäure in Betracht, ein Stoff, der einerseits ein gutes Puffervermögen aufweist und andererseits ungiftig ist. Aus der Gruppe der organischen Monomere ist vorzugsweise die Zitronensäure zu nennen; es kommen aber auch zweiwertige Säuren aus der Reihe Malonsäure, Bernsteinsäure, Glutarsäure, Adipinsäure in Betracht oder auch Weinsäure, Fumarsäure, Maleinsäure und vergleichbare Stoffe. Als besonders vorteilhaft hat sich partiell neutralisierte Zitronensäure der Formel 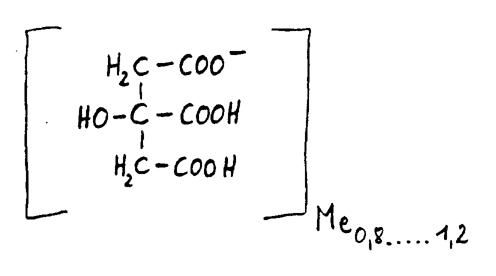 erwiesen, wobei Me ein Natrium-, Kalium- oder Ammonium-Ion bedeutet. Die erwähnten Puffer-Hilfsstoffe können dem Saugstoff in Mengen von 1 - 5 Gewichtsprozent zugesetzt werden.

Die Erfindung wird im folgenden anhand der beigefügten Zeichnung näher erläutert. Es stellen dar:

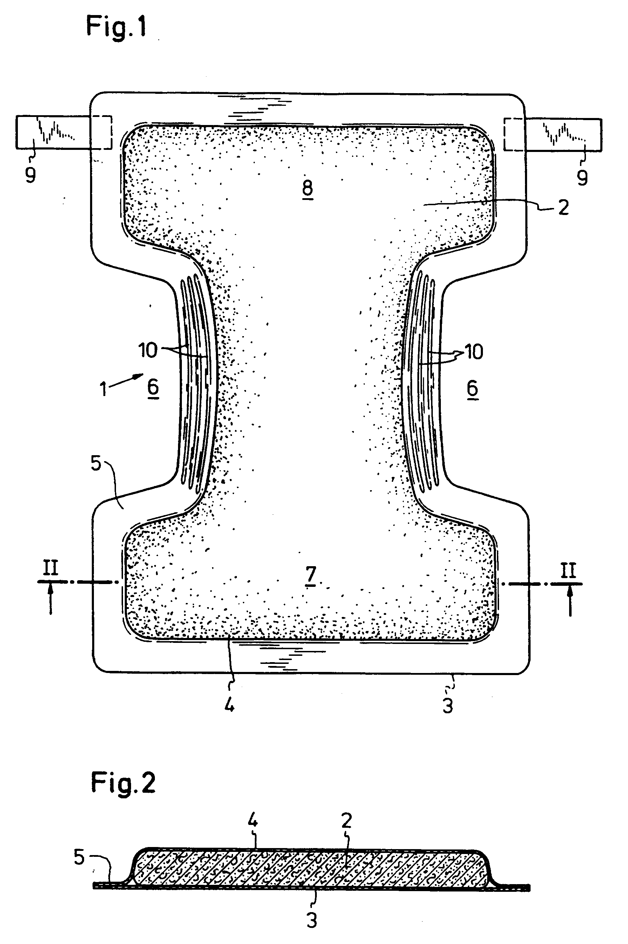

- Fig. 1: eine Draufsicht auf eine Ausführungsform einer Windel;
- Fig. 2: einen Querschnitt entlang der Linie II-II der Fig. 1.

Die in der Zeichnung dargestellte Windel ist als Ganzes mit 1 bezeichnet. Die Windel besteht im wesentlichen aus einem Saugkörper 2, bei welchem es sich um eine etwa 0,5 - 10 mm dicke Schicht von Zellstoff-Flocken handelt. Dieser Saugkörper ist auf einer Unterlage 3 angeordnet, welche aus einer Polyäthylenfolie besteht. Auf der beim Gebrauch obenliegenden Seite ist der Saugkörper mit einer Vliesstoff-Schicht 4 abgedeckt, welche die Aufgabe hat, den Saugkörper zusammenzuhalten und dessen Flusen zu verhindern, welche aber der anfallenden und vom Saugkörper aufzunehmenden Flüssigkeit keinen nennenswerten Widerstand entgegensetzt. Der Saugkörper 2 ist von dieser Vliesstoff-Schicht vollständig bedeckt; die Unterlage 3 und die Vliesstoff-Schicht 4 sind im Randbereich 5 der Windel miteinander verbunden, so daß der Saugkörper allseits vollständig von diesen Stoffen eingehüllt ist.

Die Winde 1 hat im dargestellten Ausführungsbeispiel die Form einer Sanduhr, wodurch im Mittelbereich der Winde Ausschnitte 6 entstehen, durch die die Beine des Trägers hindurchreichen können. Die Windel wird bei Gebrauch dem Träger dadurch angelegt, daß sie zwischen dessen Beinen hindurchgeführt wird und daß die breiteren Abschnitte 7 und 8 über den Bauch- bzw. Rückenbereich des Trägers gelegt werden. In diesem Zustand können die Verschlußbänder 9 am jeweils gegenüberliegenden Teil festgeklebt und die Windel auf diese Weise zu einem hosenartigen Gebilde verschlossen werden. Um einen dichten Beinabschluß zu erreichen, sind am Rande des Mittelbereiches elastische Streifen 10, beispielsweise Gummibänder, angeordnet. Diese Streifen ziehen sich soweit als möglich zusammen und legen die Beinausschnitte eng an die Haut des Trägers an.

Wesentlich im vorliegenden Zusammenhang ist naturgemäß der Saugkörper 2. Dieser Saugkörper besteht in bekannter Weise aus Zellstoff-Flocken, dem die erwähnten teilneutralisierten Quellstoffe sowie auch die ebenfalls erwähnten Hilfsstoffe beigemischt sind. Die Mischung kann dabei quasi-homogen sein; es ist aber auch möglich, nur einzelne Zonen, beispielsweise die besonders beanspruchte Mittelzone, mit diesen Stoffen auszurüsten.

### Bezugszeichenliste

- 1: = Windel
- 2: = Saugkörper
- 3: = Unterlage
- 4: = Vliesstoffschicht
- 5: = Randbereich
- 6: = Ausschnitte
- 7; 8: = breite Abschnitte
- 9: = Verschlußband
- 10: = elastische Streifen

## Patentansprüche

1. Hygienische Zellstoffartikel, wie Windel, Krankenunterlage, Damenbinde, Slipeinlage oder dergl., mit einem oder mehreren Saugkissen aus Zellstoff, mit Zusätzen an Quellstoffen und gegebenenfalls Hilfsmaterialien
sowie einer oberen flüssigkeitsdurchlässigen Abdeckung und einer unteren flüssigkeitsdichten Schicht oder Einlage,
wobei die Quellstoffe polymere organische Säuren sind und mit Alkalimetallen und/oder Ammoniak derart teilneutralisiert sind, daß sich im Zellstoff-Saugkörper bei Benetzung mit Wasser oder einer physiologischen Flüssigkeit ein pH-Wert zwischen 5,0 und 6,0, vorzugsweise zwischen 5,2 und 5,5, einstellt, dadurch gekennzeichnet,
daß die Quellstoffe Pfropfpolymere sind, mit
0,5 - 20 Gewichts-Prozent chemisch gebundenen Resten der allgemeinen Formel I,
79 - 99 Gewichts-Prozent chemisch gebundenen Resten der allgemeinen Formel II und
0,1 - 2 Gewichts-Prozent chemisch gebundenen Resten eines Vernetzers, der aus Monomeren mit wenigstens zwei olefinisch ungesättigten Doppelbindungen hervorgegangen ist;
wobei bedeuten: 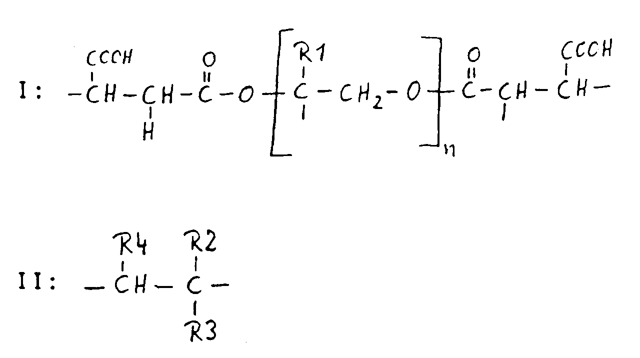
n = 2 bis 300
R1 = Wasserstoff oder Methyl
R2 = Wasserstoff, Methyl oder Ethyl
R3 = Carboxyl, Sulfonyl, Phosphonyl, gegebenenfalls verestert mit Alkanol mit 1 - 4 C-Atomen oder eine Gruppe der Formel 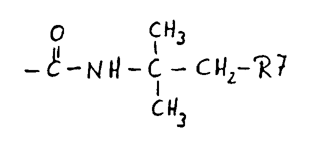 worin R7 eine Sulfonyl- oder Phosphonyl-Gruppe bedeuten,
R4 = Wasserstoff, Methyl, Ethyl oder Carboxyl.

2. Hygienischer Zellstoffartikel nach Anspruch 1,
dadurch gekennzeichnet,
daß als Hilfsstoffe im Saugkörper organische oder anorganische Monomere mit wenigstens 2 Säuregruppen vorhanden sind, die mit Alkalimetallen oder Ammoniak derart teilneutralisiert sind, daß sich bei Benetzung des Saugkörpers mit Wasser oder physiologischer Flüssigkeit ein pH-Wert zwischen 5,0 und 6,0, vorzugsweise von 5,2 - 5,5, einstellt.

3. Hygienischer Zellstoffartikel nach Anspruch 2,
dadurch gekennzeichnet,
daß als Hilfsstoff im Saugkörper 1 - 5 Gewichtsprozent partiell neutralisierte Zitronensäure der Formel 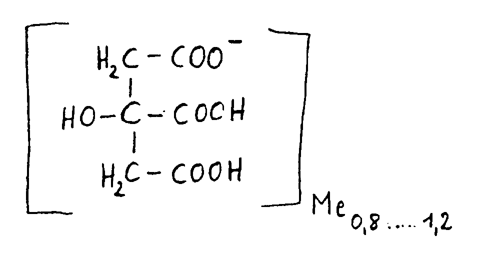 vorhanden ist, wobei Me ein Natrium-, Kalium- oder Ammonium-Ion bedeutet.

## Claims

1. Sanitary cellulose article such as a diaper, sick-sheet, sanitary towel, protection pad or the like, comprising one or a plurality of absorbent cellulose paddings, additions of swelling agents and possibly auxiliary agents, as well as an upper liquid-permeable cover and a lower liquid-proof layer or insert,
the swelling agents being polymeric organic acids and being partially neutralized by means of alkali metals and/or ammonia such that a pH ranging between 5.0 and 6.0, preferably between 5.2 and 5.5, ensues in the absorbent cellulose body when the latter is wetted with water or a physiological liquid,
characterized in that
the swelling agents are graft polymers having
0.5 - 20 percent by weight of chemically combined residues of the general formula I,
79 - 99 percent by weight of chemically combined residues of the general formula II, and
0.1 - 2 percent by weight of chemically combined residues of a cross-linking agent originating from monomers having at least two olefinically unsaturated double bonds;
I representing: 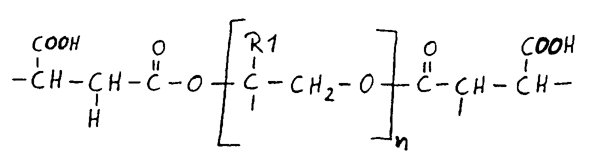
II representing: 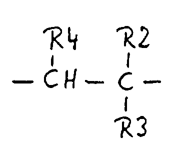
n = 2 to 300
R1 = hydrogen or methyl
R2 = hydrogen, methyl or ethyl
R3 = carboxyl, sulphonyl, phosphonyl, possibly esterified with alkanol having 1 - 4 atoms or a group of the formula 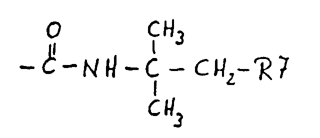 R7 representing a sulphonyl or phosphonyl group,
R4 = hydrogen, methyl, ethyl or carboxyl.

2. Hygienic cellulose article according to claim 1,
characterized in that
organic or inorganic monomers having at least 2 acid groups exist as auxiliary agents in the absorbent body, which are partially neutralized by alkali metals or ammonia such that a pH ranging between 5.0 and 6.0, preferably between 5.2 and 5.5, ensues when the absorbent body is wetted with water or a physiological liquid.

3. Sanitary cellulose article according to claim 2,
characterized in that
1 - 5 percent by weight of partially neutralized citric acid of the formula 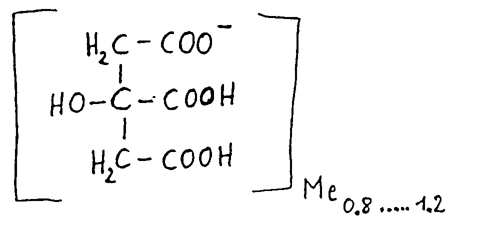 exists as an auxiliary agent in the absorbent body, Me representing a sodium, potassium or ammonium ion.

## Revendications

1. Article hygiénique en cellulose, comme une lange, alèze, serviette hygiénique, un protège-slips ou analogue, avec un ou plusieurs coussins absorbants en cellulose, avec des additifs en agents gonflants et, s'il y a lieu, des auxiliaires
ainsi qu'un recouvrement supérieur perméable aux liquides et une couche inférieure imperméable aux liquides ou un insert inférieur imperméable aux liquides,
les agents gonflants étant des acides polymères organiques et étant partiellement neutralisés à l'aide de métaux alcalins et/ou de l'ammoniac de telle sorte qu'un pH entre 5,0 et 6,0, de préférence entre 5,2 et 5,5, se manifeste dans le corps absorbant en cellulose lors d'un mouillage par de l'eau ou un liquide physiologique,
caractérisé en ce que
les agents gonflants sont des polymères greffés avec
0,5 - 20 % en poids de restes combinés chimiquement de la formule générale I,
79 - 99 % en poids de restes combinés chimiquement de la formule générale II, et
0,1 - 2 % en poids de restes combinés chimiquement d'un réticulant provenant de monomères avec au moins deux liaisons doubles insaturées à l'oléfine;
I représentant: 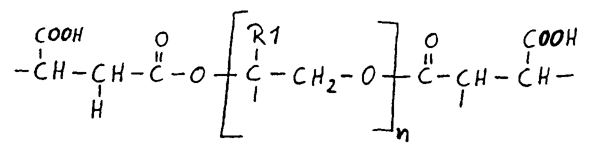
II représentant: 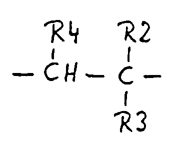
n = 2 à 300
R1 = hydrogène ou méthyle
R2 = hydrogène, méthyle ou éthyle
R3 = carboxyle, sulfonyle, phosphonyle, s'il y a lieu estérifiés d'alkanol à 1-4 atomes C, ou un groupe de la formule 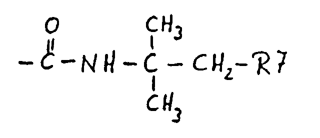 R7 représentant un groupe de sulfonyle ou phosphonyle,
R4 = hydrogène, méthyle, éthyle ou carboxyle.

2. Article hygiénique en cellulose selon la revendication 1,
caractérisé en ce que
des monomères organiques ou inorganiques avec au moins 2 groupes d'acides se trouvent dans le corps absorbant comme auxiliaires, qui sont partiellement neutralisés à l'aide de métaux alcalins ou de l'ammoniac de telle sorte qu'un pH entre 5,0 et 6,0, de préférence de 5,2 à 5,5, se manifeste lors d'un mouillage du corps absorbant par de l'eau ou un liquide physiologique.

3. Article hygiénique en cellulose selon la revendication 2,
caractérisé en ce que
1 - 5 % en poids d'acide citrique partiellement neutralisé de la formule 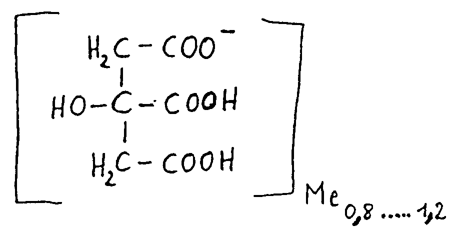 se trouve comme auxiliaire dans le corps absorbant, Me représentant un ion de sodium, de potassium ou d'ammonium.
